(19) **Europäisches Patentamt / European Patent Office / Office européen des brevets**

(11) **EP 4 736 827 A1**

(12) # EUROPEAN PATENT APPLICATION

(43) Date of publication:
06.05.2026 Bulletin 2026/19

(21) Application number: 24210515.3

(22) Date of filing: 04.11.2024

(51) International Patent Classification (IPC):
*A61F 13/505* (2006.01)    *A61F 13/49* (2006.01)
*A61F 13/533* (2006.01)    *A61F 13/534* (2006.01)
*A61F 13/53* (2006.01)

(52) Cooperative Patent Classification (CPC):
**A61F 13/505; A61F 13/49003; A61F 13/533;
A61F 13/53436;** A61F 2013/530313

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Essity Hygiene and Health Aktiebolag
405 03 Göteborg (SE)**

(72) Inventors:
• KNÖS, Anna
405 03 GÖTEBORG (SE)
• BLOMSTRÖM, Philip
405 03 GÖTEBORG (SE)
• FLODIN, Alice
405 03 GÖTEBORG (SE)

(74) Representative: **Nederlandsch Octrooibureau**
P.O. Box 29720
2502 LS The Hague (NL)

(54) **ABSORBENT ASSEMBLY FOR WASHABLE ABSORBENT UNDERGARMENT**

(57) A washable integrated absorbent assembly (10) for a washable absorbent undergarment (1), the assembly extending in a longitudinal direction and having a transverse direction. The assembly comprises an absorbent core layer of knitted or woven material connected to one or more further layers and the core layer is provided with one or more stress-release zones to facilitate stretching of the core layer.

# Fig. 4

## Description

### Field

The present disclosure relates to washable absorbent undergarments comprising an integrated absorbent assembly having an absorbent core layer. The disclosure also relates to such washable absorbent assemblies. Such articles and assemblies are intended for reusable sanitary use in absorbing body fluids such as urine and vaginal fluids. The disclosure relates in particular to an absorbent assembly having improved flexibility.

### Background

A washable absorbent undergarment may be worn for the purpose of absorbing body fluids such as urine and vaginal fluids. Such garments form an environmental and economical advantageous alternative to disposable sanitary napkins and disposable absorbent undergarments. A washable absorbent undergarment typically comprises fabric panels of woven or knitted materials. Conventionally such a washable absorbent undergarment will also comprise an integral absorbent assembly located in the crotch region of the wearer when the undergarment is worn, sometimes referred to as the gusset. After use the undergarment is washed or laundered before reuse. An example of such a washable absorbent undergarment is found in WO2023169665A1.

Depending upon the intended use, the absorbent assembly may vary in size and construction. It may extend over a smaller or larger area of the garment, e.g. depending on whether it is intended for daytime or nighttime use. It may also be thicker or thinner, with additional wicking, distribution, barrier, and absorbing layers. Nevertheless, in addition to being functional from an absorbing perspective, the resulting undergarment must inspire confidence and be comfortable to wear for prolonged periods as well as providing a good fit and being aesthetically pleasing.

The selection of materials for incorporation into the absorbent assembly is one of the major challenges in developing such undergarments. Natural materials having adequate absorbent capacity are available but are not easily integrated into a garment, while maintaining the levels of comfort, confidence and discretion expected by today's users. It would thus be desirable to improve the efficacy of materials intended for use in the absorbent assembly.

### Summary

This object may be at least partially achieved with an absorbent assembly in accordance with claim 1 and a washable absorbent undergarment according to claim 13. Further embodiments are set out in the dependent claims and in the following description.

The disclosure thus concerns an integrated absorbent assembly or gusset for a washable absorbent undergarment. The absorbent assembly extends in a longitudinal direction and has a transverse direction and further comprises an absorbent core layer of knitted or woven material connected to one or more further layers. The core layer is provided with one or more stress-release zones, which facilitate stretching of the core layer. As a result of the stress-release zones, materials may be used for the core layer, which would be otherwise too stiff or inextensible for integration or connection with the further layers. It will be understood that an absorbent assembly, located in the crotch region of a garment must exhibit a minimum of flexibility for it to comfortably conform to the body of a user. Without adequate flexibility, the garment or at least the absorbent assembly will be uncomfortable and may leak due to failure to remain in the intended position. The flexibility of the absorbent assembly will be determined by all the layers making up the assembly and the garment. Nevertheless, it will be understood that it will be the stiffest layer of the combination that will be the limiting factor. By providing stress-release zones in the core layer of the assembly, it may be avoided that this layer is the limiting factor, even if relatively inflexible materials are used.

The stress-release zones may extend at least partially in the transverse direction to facilitate and/or enhance the stretchability of the core layer and absorbent assembly in the longitudinal direction. This is the primary direction for which the assembly should be able to stretch during use although it will be understood that diagonal stretch or shear is also important. This can thus be enhanced by the presence of the stress-release zones extending at least partially in the transverse direction. In the following, stretchability will be used to refer to extension in a given direction whereas flexibility will be used more generally to refer to extensions in any combinations of directions.

The core layer may comprise a plurality of separate segments. The stress-release zones may be the gaps or separations that extend between adjacent segments. The segments may be separately produced and individually connected to the further layers. Alternatively, the core layer, may be produced as an item and subsequently cut into the segments, prior or after connecting it to the further layers. The segments may abut one another in the relaxed state of the garment. On stretching the garment or the absorbent assembly, the segments may move apart as the further layers stretch. Alternatively, the segments may initially be spaced from each other.

The stress-release zones may also or alternatively comprise incisions extending only partly across the core layer to divide the core layer into interconnected regions. The core layer may thus remain as a single element with all regions being interconnected. It is also possible that individual segments of the layer may be provided with incisions. The incisions may merely cut through the material or may also remove material, leaving an opening. In general, the incisions will also pass completely through the core layer from an inner side of the material to an outer side of the material. It is however not excluded that a number of core layers are provided and an incision in one core layer need not be aligned with an incision in another core layer. An incision is per definition not the same as an opening or a hole inherent in a material layer and that is being made during the manufacturing of the material, such as tuck stitches made during a knitting process, eyelet weaving or a spacer fabric with openings. Incisions is a subsequent operation made in an absorbent core layer to introduce stress release zones to facilitate stretching of the core layer and to accommodate its stretchability to further layers in the absorbent assembly.

Furthermore, each incision may extend between 10 % and 90 % across the width of the core layer in the transverse direction, such as 10 to 50%. In the present context, the term incision is used to denote a cut that does not extend to the edge of the material at either of its ends. It will be understood that if a cut extends across 100 % of the core layer, then separate segments ensue as discussed above. Incisions that extend only a small distance across the core layer may have less effect on the stretchability than longer incisions. Nevertheless, multiple transverse incisions may be aligned with each other to increase the stretchability. The lateral edges of the core layer may be continuous i.e. without incisions. As will be discussed further below, this allows the core layer with incisions to be connected to the further layer at the lateral edges and/or be connected to the garment.

The core layer of the absorbent assembly may be provided with just 1 or as many as 100 incisions. Large numbers of incisions may be arranged in various patterns to optimise stretchability or flexibility. There may be from 2 to 10 separate incisions.

Depending on the number of complete or partial separations, the core layer may comprise from 2 to 20 separate segments, such as 3 to 5 separate segments or partly interconnected regions. It will be understood that more segments or regions may increase the stretchability but may also reduce the integrity of the core layer and make it more difficult to connect to the remainder of the assembly or the garment.

The stress-release zones may be linear e.g. extending only in a single direction such as the transverse direction. The stress-release zones may be non-linear. This may include curved, angled, chevron or zig-zag zones or incisions.

The core layer may comprise any suitable washable knitted or woven material for which the provision of stress-release zones is appropriate. In those cases where stress-release is achieved by incisions wherein the core layer is cut either fully or partially, materials may be desirable that maintain their integrity despite cutting. A double-knit material may be used, which in the present context is intended to denote a fabric created by interlocking two layers of fabric during the knitting process. The knitting process involves using two sets of needles that work together to create this double layer of fabric. The layers are tightly knit together, resulting in a fabric that is not only thicker, but also more durable than other types of knit fabrics. Due to its double-layer construction, double knit fabric retains its shape well and is resistant to wrinkles. The core layer may be of an interlock knit fabric. Interlock knit fabric is a variation of rib knit. It has two rows of stitches created one behind the other, using two rows of needles that cross over each other and the rows become "interlocked" as the fabric is knitted. Interlock knit fabric is a specific form of double-knit fabric. The interlocked fabric prevents runs and does not unravel or curl at the edges allowing incisions to be made without the material unraveling.

The core layer may further be defined by various properties or combinations of properties and may make use of other knitting variations. It may comprise a double-sided weft knit, which may include the interlock fabric mentioned above or it may be provided e.g. as a single-sided or double-sided terry finish. Such terry finishes have been shown to have good absorption capacity. The core layer material may have a base weight of 180-600 gsm, such as 300-500 gsm. This basis weight may be achieved by a single layer of core material although it is not excluded that multiple core layers may be provided to increase the basis weight.

Furthermore, various materials and yarns may be implemented in the core layer, including any material capable of absorbing fluid, such as microfibre or polymer fibres, hydrophilic fibres, absorbent fibres and the like. The material of the core layer may be predominantly of natural origin including naturally derived fibres but may include small amounts of synthetic materials. In the present context, naturally derived fibres include fibres that have been synthesised from bio-based materials such as cellulose. Such materials include e.g. regenerated cellulose, viscose, lyocell, rayon, Tencel and modal. Natural fibres further include cotton, linen, wool, silk, hemp, jute, bamboo, ramie, coir, banana and the like. The core may comprise between 20% and 100% natural fibres, such as between 40% and 100% natural fibres, such as between 60% and 100% natural fibres with the remainder being synthetic fibres. Alternatively, the core may comprise between 20% and 100% naturally derived fibres, such as between 40% and 100% naturally derived fibres, such as between 60% and 100% naturally derived fibres with the remainder being other natural fibres and/or synthetic fibres. The core layer should be washable.

The yarns forming the core layer may have any appropriate construction or weight to achieve the desired absorption capacity. These may be spun yarns of short staple fibres and may be single-ply or multi-ply. The yarns may be relatively fine to favour a close knit or weave that will not easily unravel.

The absorbent assembly itself is a multi-layer structure of which the core layer forms one of the layers having the primary absorbing function. As mentioned elsewhere, there may be one or more core layers having the same or similar construction and the same or similar function. Each may be provided with stress-release zones, it being understood that the least stretchable layer will dictate the final property of the assembly in this respect. These may be overlaid on the garment facing side and/or on the wearer facing side of the assembly by the further layers, having other functions and properties.

The further layers may comprise a wearer facing top layer. The top layer may comprise a water-permeable material allowing the body fluids to migrate to the underlying core. The top layer may be constructed of any suitable washable fabric, including natural and/or naturally derived fibres selected from the group consisting of cotton, wool, silk, cellulose, regenerated cellulose, rayon, viscose, modal, lyocell, Tencel, bamboo, hemp, flax, ramie, coir or banana. Alternatively, the top layer may be constructed of synthetic fibres selected from the group consisting of polyamide, acrylic, polyester, or elastane, such as a mixture of polyester and elastane. Further, the top layer may be constructed of a blend or a mixture of naturally derived and/or synthetic fibres. The materials used for construction of the top layer should be soft and non-irritating to the skin and be readily penetrated by any body fluids. The top layer is washable and may be a knitted material. The top layer may comprise between 20% and 100% natural or naturally derived fibres, such as between 40% and 100% natural or naturally derived fibres, such as between 60% and 100% natural or naturally derived fibres. The top layer may have a base weight of 80-200 gsm.

The further layers may comprise a washable moisture distributing or wicking layer, which may be provided directly underneath the top layer. The wicking layer may have wicking features to allow the moisture to spread away from the wearer and into the core. Wicking enables an efficient spread of the moisture, allowing the moisture to be received into a wider area of the underlying core layer and absorbent assembly. This feature is particularly relevant for users suffering from stress incontinence as spurts of urine may cause the core layer to be saturated rapidly at the point of impact. This function is also very important when the core layer comprises isolated or partially isolated segments or regions, whereby wicking and fluid transmission between segments/regions is reduced. By spreading the volume of the fluid over a wider receiving area in the assembly, the wicking features of the wicking layer increase the overall absorptive capacity of the core layer. The wicking layer may be constructed of any suitable washable fabric, including natural and/or naturally derived fibres or mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the wicking layer may be constructed of synthetic fibres or mixtures thereof selected from the group consisting of polyamide, acrylic, polyester, or elastane. Further, the wicking layer may be constructed of a blend or a mixture of natural, naturally derived and/or synthetic fibres. According to the present disclosure, the wicking layer should be launderable. The wicking layer may comprise between 20% and 100% natural or naturally derived fibres, such as between 40% and 100% natural or naturally derived fibres, such as between 60% and 100% natural naturally derived fibres. The basis weight of the wicking layer may be 180-250 gsm.

The further layers may comprise a washable garment facing moisture barrier layer. The moisture barrier layer functions to prevent moisture from penetrating through the core layer to the fabric panels of the undergarment. The moisture barrier layer may be an extruded layer. Suitable moisture impermeable materials may include thermoplastic films, such as polyurethane or the like. Alternatively, it may comprise a weave or knit. Certain filaments and yarns are naturally hydrophobic and a closely woven or knit structure can thus achieve significant water repellence without need for additional coatings. The moisture barrier layer may also comprise a moisture impermeable material laminated or coated onto a woven or knitted material. The moisture barrier layer may have a surface weight of between 50 and 300 gsm, such as between 75 gsm and 200 gsm. Although the moisture barrier layer may be impervious to liquid water, the moisture barrier layer may be breathable. It may have a breathability or moisture vapor transmission rate (WVTR) of more than 500g. WVTR is measured by the rate at which water vapor passes through, in grams of water vapour per square meter of fabric per 24-hour period (g/m2/d), often abbreviated to just "g".

As a result of the stress-release zones, the core layer may exhibit greater stretchability than would have been the case without the stress-release zones. The material of the core layer may be considerably less stretchable than other layers of the absorbent assembly such as the moisture barrier layer. With the inclusion of the stress-release zones, the resulting assembly may become more stretchable, and the core layer may no longer be a limiting factor on the resilience or flexibility of the gusset.

In particular, core layer material may be employed that, without stress release, would be at least twice as inelastic as any of the other layers of the absorbent assembly or garment. In other words, the materials of the other layers may exhibit stretch that is twice or more that of the core layer. It will be understood that these values are given for the basis weight as employed in the assembly and undergarment.

Stretchability may be measured in conventional manner using a tensile tester e.g. from the Instron, Lloyd or Zwick companies based on a 40 mm width sample and a 4 N weight for a sample length of 40 mm (clamping distance). The result is expressed as percentual stretch at a force of 1 N per cm of sample width. Materials for the core layer may be used exhibiting a stretch of just 10% to 30%, without stress relief. The other layers of the assembly and garment may all individually be more stretchable than the core layer material, at least in the longitudinal direction. In particular, the other layers may all exhibit stretchability of greater than 50% or greater than 60% or greater than 70% in the longitudinal direction. The other layers of the assembly may all collectively be more stretchable, at least in the longitudinal direction, than the core layer material without stress relief.

Other layers may also be present, or the above further layers may be further complemented with other functionalities. Such layers or functionalities may include anti-microbial, anti-odour, antifungal and the like.

The core layer may be connected to the one or more further layers in any suitable manner. The core layer is connected to the one or more further layers at its side edges, which may also correspond to the edges of the assembly. This may be sufficient in cases where the core extends the full width of the assembly without interruption. The core layer may be connected to the one or more further layers only at its longitudinal side edges. It may also or alternatively be connected to the one or more further layers at its transversal ends. In cases where the core layer is interrupted by cuts or incisions between the edges of the assembly, it may be necessary to connect the core layer to the one or more further layers at other locations over its surface. The layers may be connected by stitches, staples, adhesive, welding, tape or the like. The core layer may be connected to just one of the further layers, e.g. to the top layer or wicking layer that is at the wearer facing side. Alternatively, it may be connected to further layers on either side of it. The core layer may be connected to all of the layers of the assembly and/or to fabric panels of the undergarment. In this context, connected is intended to specifically include the case where the further layer to which the core layer is connected is a fabric panel of the garment.

The assembly may have an area adapted to the particular size of undergarment and intended use. The assembly may have an area of at least 10 cm$^2$, such as between 10 cm$^2$ and 400 cm$^2$, or between 50 cm$^2$ and 100 cm$^2$. The core layer may have a similar area and may be co-extensive or substantially co-extensive with the assembly over the whole area of the assembly. The core layer may occupy at least 40 % of the area of the assembly or more than 60 % or more than 70% or more than 90 % or more than 95% of the area.

The disclosure also relates to a washable absorbent undergarment comprising one or more woven or knitted fabric panels and a washable integrated absorbent assembly as described above and hereinafter located in a crotch region.

As used herein, the term "absorbent undergarment" refers to garments that are worn and intended to be placed against the skin of the wearer with the intended function of absorbing and containing body fluids such as urine and vaginal fluids including menstrual fluid, without significant visible external trace. The undergarment may for example be underwear, underpants, a panty, or swimwear of fabric. The undergarment is intended for teenage and adult users. The undergarment may be designed for male or female use. It will be understood that most conventional garments will have a limited degree of absorbency but that for conventional undergarments the containment of body fluids is not intended. An unexpected leakage of any significant volume would thus be perceptible externally either as a damp patch or discoloration that could penetrate through an outer garment.

The absorbent undergarment and the absorbent assembly according to the present disclosure is washable, i.e. intended to be laundered or otherwise restored after use for repeated reuse as a sanitary article. By "washable" it is meant that the absorbent undergarment comprises woven or knitted fabric and may be cleaned by laundering. The absorbent undergarment may thus be subjected to an aqueous solution containing detergent without losing its structural features. This aqueous solution may be heated as part of the laundering process, e.g. to 30°C or 60°C. The term is also intended to mean that the absorbent undergarment can be washed and reused more than once. Washable may be understood as being suitable to undergo at least 10 washing cycles at a temperature of at least 30°C, without disintegrating. During the washing, the absorbent assembly releases the absorbed fluids, thereby regenerating the absorptive functioning, enabling the absorbent assembly to be reused for the same purpose again. A disposable absorbent article is not constructed to be washable and reusable and is thus for single use only. A disposable absorbent article is made up of materials such as nonwoven materials and laminates, plastic films, superabsorbent polymer particles and cellulose fibres.

The term "fabric" as used in the present disclosure may refer to single or multiple layers of a flexible planar substance constructed from solutions, fibers, yarns, fabrics, or any combination. Fabrics include knitted or woven materials. The term textile refers to any flexible material that is composed of thin films or of fibers, yarns, or fabrics or products made of films, fibers, yarns, or fabrics. Although other terms like material, fabric, and cloth are synonyms, textile is most used in the global textile complex. Fiber is any substance, natural or manufactured, with a high length-to-width ratio with suitable characteristics for processing into fabrics and is the smallest component, hairlike in nature, that can be removed from a fabric. Fibers make most fabrics. A yarn is a grouping of fibers that is twisted or laid together to form a continuous strand that can be made into a textile fabric.

The assembly may be permanently attached to at least one of the one or more woven or knitted fabric panels. By "permanently attached", is meant that no portion is intended to be separated from the body fabric during, or after use. All portions of the assembly are thus intended to be laundered together with the garment. The assembly may alternatively be configured as a pad that is adapted for detachable connection to the panels of an undergarment. Thereto the assembly may comprise an adhesive layer or a hook patch on its garment facing surface or any other suitable manner of connection. Alternatively, the garment may be provided with a suitable pocket or recess for receiving the assembly.

The assembly may be attached to the fabric panels of the undergarment over its whole surface e.g. by lamination or adhesive. The absorbent assembly may not be directly bonded to the fabric panels but instead may be attached to the fabric via auxiliary parts such as edging strips or reinforcement patches. The absorbent assembly may be attached to the one or more fabric panels by one or more bonding members. These may include one or more of a front bond member, a rear bond member and side bond members.

The undergarment may be of any suitable form and reference above and in the following to 'garment' is not intended to mean other than an undergarment i.e. an innermost garment intended for being in contact with the body of a user. The garment has a waist opening and two leg openings. It is however not excluded that closure elements e.g. between the waist opening and the leg openings may be provided, allowing easy removal, or donning of the garment. The undergarment may have various designs, such as briefs, boxer, hipster, high waist, string, Brazilian, or other suitable and conventional undergarment designs. The fabric panels may form a front region, a back region and a crotch region extending between the front and back regions, the front region and the back region being joined at the sides such that the undergarment forms a waist opening and a pair of leg openings. A central longitudinal axis of the undergarment is defined along the one or more fabric panels of the undergarment from the back region and towards the front region and a transversal crotch axis of the undergarment is defined in a direction extending between the leg openings. The transversal crotch axis may be located at the narrowest point of the garment and/or may be located at the mid-point of the central longitudinal axis. The transversal crotch axis divides the crotch region into a front crotch region extending longitudinally between the transversal crotch axis and a front end of each leg opening, and a rear crotch region extending longitudinally between the transversal crotch axis and a rear end of each leg opening. The transversal crotch axis is perpendicular to the central longitudinal axis and the core and further layers are superimposed along a height axis, that is perpendicular to the longitudinal axis and the transversal crotch axis.

The assembly may be located in the crotch region. It may also be located only in the crotch region i.e. it may not extend into the front region and back regions, which together form the waist region. The assembly nay be located predominantly in the front crotch region. The assembly may be substantially symmetrical along the central longitudinal axis.

The shape and size of the assembly may be such that at least a crotch region of said undergarment is covered during use. The crotch region is a surface section having a size and shape to cover some or all of the genital area of a user, i.e. a surface section shaped to fit in between the legs of a user, that has a length sufficient to extend at least from the mons pubis to the perineum and a width that varies along the length. The crotch region thus has a width substantially corresponding to a transversal width in between leg openings of an undergarment. Thus, the entire crotch region of the undergarment is protected from soiling by the assembly.

The area of the garment in which the assembly is present may be adapted according to the particular garment and the intended use. The assembly may cover an area of at least 10 cm$^2$ of the garment, such as between 10 cm$^2$ and 400 cm$^2$, or between 50 cm$^2$ and 100 cm$^2$.

The assembly may be of any appropriate shape according to the intended use and the shape of the undergarment. It may be dog-bone shaped, having curved sides, and slightly enlarged ends. It may be four-sided, having a front edge, a rear edge and two side edges. The side edges may be positioned to be coincident with respective leg openings. In this context, "coincident" is intended to mean substantially aligned with and adjacent to the side edges, within the bounds of normal manufacturing practices. The assembly or parts thereof may wrap around the side edges in a seam or vice-versa. The assembly may also end just short of the side edges and be covered by an edging strip. The absorbent core layer may have the same size and shape as the assembly or may have the same shape but be marginally smaller in size.

The assembly may be rectangular. In this context, "rectangular" is intended to denote that the assembly has an outer contour that is a four-sided shape, having two pairs of sides that are generally parallel to one another. The rectangular assembly or its components can be easily cut multiple times from a main material source (often a large sheet or roll of fabric or foil material), with a single cutting line forming two edges of adjacent components. Thus, the use of rectangular shaped assemblies reduces the amount of cutting and minimizes waste. Furthermore, the cutting of rectangular contours from a main material source allows for multiple components to be cut directly adjacent to one another, without having to account for a positioning direction of each individual component in the manufacturing line. Thus, no energy is wasted during production to account for variation in direction of each component.

At least 75% of the total length of the edges of the at least one rectangular shaped assembly may be straight edges. Thus at least 75% of the outer contour of the rectangular shaped assembly conforms to the rectangular shape, thereby limiting the amount of material wasted, while allowing for some minimal shape variations in core layer that are required to assure wearer comfort and prevent the article from bulging during use.

The one or more further layers may all be substantially equal in size and shape and may all have an outer contour that forms an outer contour of the assembly. In this manner, protection against leakage is available over the same area that is also directly visible to the user, giving a good indication to the user of the leakage protection provided by the assembly. The layers need not be co-extensive. A top layer may extend beyond the moisture barrier layer e.g. to connect to the fabric panels of the garment.

## Brief description of the drawings

A washable absorbent undergarment according to the present disclosure will be described by way of example, with reference to the attached drawings, in which:

Fig. 1 shows a front view of an exemplary absorbent undergarment comprising an absorbent assembly;
Fig. 1A shows a detail of the garment of Fig. 1;
Fig. 2 shows a top view of the undergarment of Fig. 1 in a flat laid-out state with the joints between the rear region and the front region removed;
Fig. 3 is a schematic cut-away view of a portion of the absorbent undergarment of Fig. 1;
Fig. 4 is a cut-away perspective view through the undergarment of Fig. 1 in the crotch region;
Fig. 5 is a plan view of the assembly of Fig 4;
Fig. 6 is a plan view of the assembly of Fig. 5 under an applied force F;
Fig. 7A-D are plan views of alternative assembly arrangements; and
Fig. 8A-C are plan views of further alternative assembly arrangements.

## Description

Different aspects of the present disclosure will be described more fully hereinafter with reference to the enclosed drawings. The absorbent undergarment disclosed herein can, however be realized in many different forms, such as different sizes and absorption levels, and should not be construed as being limited to the aspects set forth herein. In all figures of the following detailed description, the same reference numerals will be used to indicate the same elements.

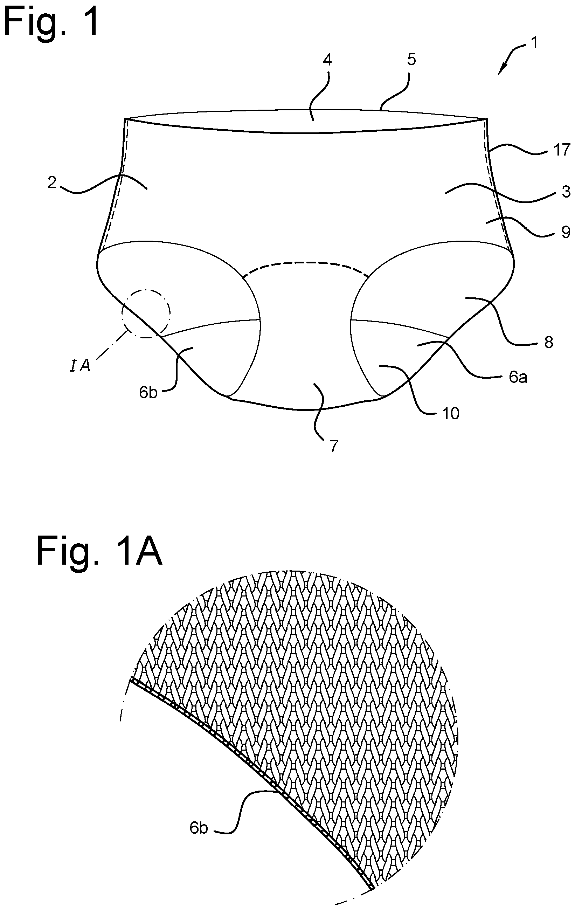

Fig. 1 illustrates as an example a washable and reusable absorbent undergarment 1. As outlined in the above, the undergarment 1 as proposed herein may however have various designs, such as briefs, boxer, hipster, high waist, string, Brazilian, or other suitable undergarment designs. The undergarment 1 may be designed for male or female use.

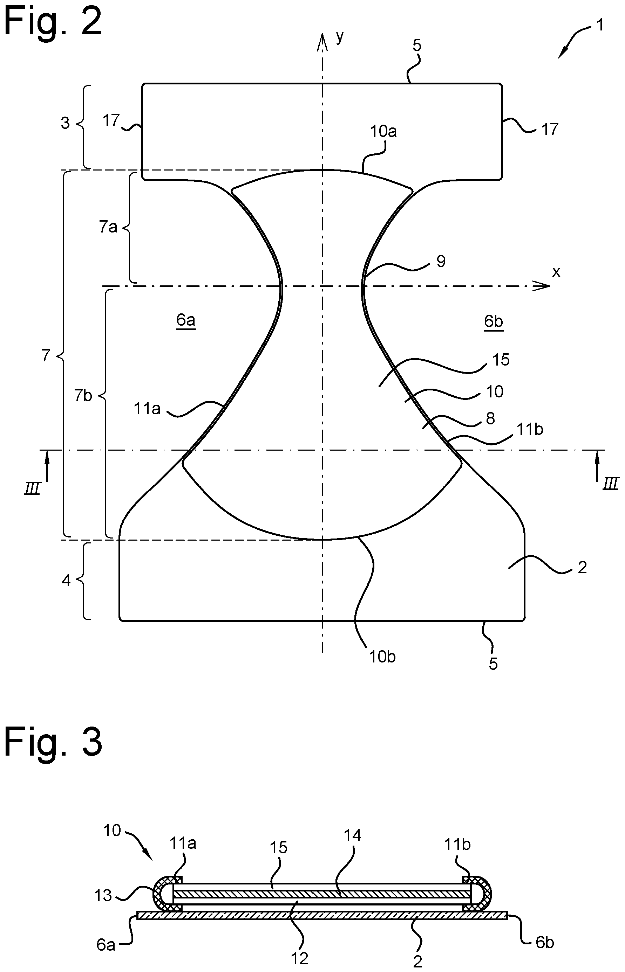

The undergarment 1 comprises one or more knitted or woven fabric panels 2 forming a front region 3, a back region 4 and a crotch region 7 extending between the front and back regions 3, 4. The front region 3 and the back region 4 are joined such that the undergarment 1 forms a waist opening 5 and a pair of leg openings 6a, 6b. As such, the front region 3 will be visible from the front of the user when the undergarment 1 is worn, and the back region 4 will be visible from the back of the user when the undergarment 1 is worn. The undergarment of the Fig. 1 and Fig. 2 example comprises one fabric panel 2, which is cut to form the front region 3, the back region 4 and the crotch region 7. However, in other variants, a plurality of fabric panels may be joined to form the front region 3, the back region 4 and the crotch region 7. For example, the undergarment 1 may comprise a front panel, a back panel, and an intermediate panel. The one or more fabric panels may all comprise the same fabric, giving the undergarment a unitary appearance, or the one or more panels may comprise different fabrics, for example to provide aesthetically pleasing undergarments comprising e.g. lace fabric.

The fabric of the one or more fabric panels 2 may be any suitable fabric, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir, or banana. Alternatively, the fabric may be constructed of synthetic fibres or mixtures thereof selected from the group consisting of polyamide, acrylic, polyester. Further, the fabric may be constructed of a blend or a mixture of naturally derived and/or synthetic fibres. The fibres may be recycled fibres. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric may be breathable to allow vapor to escape from the wearer's skin and from the absorbent structure.

As illustrated, the body fabric is a single layer of interlock knitted fabric that terminates at the waist opening 5, without any waist band or additional elastic. There is also no seam, elastic or stitching around the leg openings 6a, 6b as illustrated in Fig 1A. A characteristic of interlock knitted fabric is that it can be cut, and the cut edge is stable against fraying without requiring any form of seam. It will nevertheless be understood that the absorbent undergarment 1 may comprise a waist band, arranged along the waist opening 5 of the absorbent undergarment 1, which may be elasticated and/or provided with a frictional inner surface.

To join the front region 3 and the back region 4 to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or conventional mechanical bonds, such as stitching techniques. As illustrated in Fig. 1, the fabric panel 2 has an outside surface 9 facing away from the wearer and an inside surface 8 facing in a direction towards the wearer. An absorbent assembly 10 is located in the crotch region 7 at the inside surface 8.

In Fig. 2, the absorbent undergarment 1 of Fig. 1 is shown in a flat laid-out state, where the side joints 17 of the undergarment 1 are removed. As seen in this laid-out state, a central longitudinal axis y is defined along the one or more fabric panels 2 and in a direction from the rear side 4 of the undergarment 1 towards the front side 3 of the undergarment 1. In Fig. 2, the absorbent undergarment 1 is shown from a wearer- facing side of the article 1. As illustrated in Fig. 1, the one or more fabric panels 2 will have an exterior side 9 facing away from the wearer, and an interior side 8 facing in a direction towards the wearer. Fig. 2 thus displays the undergarment 1 in a flat laid-out state as seen from the interior side 8. Further, a transversal crotch axis x of the undergarment 1 is defined in a direction extending between the leg openings 6a, 6b, the transversal crotch axis x being perpendicular to the central longitudinal axis y at the crotch, being the position of minimum distance between the leg openings 6a, 6b.

The assembly 10 comprises a wearer facing top layer 15 that visibly illustrates to a user the extent of the assembly 10. The transversal crotch axis x divides the crotch region 7 into a front crotch region 7a extending longitudinally forwards from the transversal crotch axis x to a front edge 10a of the assembly 10, and a rear crotch region 7b extending longitudinally between the transversal crotch axis x and a rear edge 10b of the assembly 10. The longitudinal extension of the assembly 10 may depend for example on the design of the undergarment 1.

The assembly 10 thus defines the crotch region 7, being generally at a location where the need for absorbance is most prevalent. For undergarments intended primarily for night-time use, the assembly 10 may extend further rearwards and more of the assembly 10 may be located to the rear of the transversal crotch axis x extending further upwards towards the waist opening 5.

As exemplified by the illustrated undergarment 1, the assembly 10 also forms a pair of side edges 11a, 11b. Each side edge 11a, 11b is at least partly directed towards a respective leg opening 6a, 6b. Each side edge 11a, 11b, thus connects the front edge 10a and the rear edge 10b. Each side edge 11a, 11b is arranged to follow the contour of the respective leg opening 6a, 6b of the undergarment 1.

The skilled person will recognise that the shape of the assembly 10 may alternatively be adapted to various absorption needs and to various designs of the undergarment 1, to provide sufficient absorption and satisfactory fit. For example, the side edges 11a, 11b may be straight or may comprise concave portions, as seen towards the central longitudinal axis y. Thus, the assembly 10 may be better adapted to fit between the wearer's legs. The front edge 10a of the assembly 10 may be curved convex as shown. This may be beneficial for the fit of the undergarment to the body and contribute to the avoidance of unwanted creases in the fabric panels. The front edge 10a may also be straight to reduce production and material costs. The same may apply to the rear edge 10b.

Fig. 3 shows a cross-section through the rear crotch region 7b, taken in the direction III-III in Figure 2. The fabric panel 2 can be seen to comprise a single layer of interlock knitted fabric without any seam or other reinforcement at the leg openings 6a, 6b. Beneath the top layer 15 is an absorbent core layer 14 and a moisture barrier layer 12. The core 14 layer is co-extensive with the barrier layer 12, and the top layer 15. The top layer 15, the core layer 14 and the barrier layer 12 are connected around the side edges 11a, 11b and the front and rear edges 10a, 10b by single sided adhesive tape 13. The assembly 10 is attached to the fabric panel 2 by adhesive. It will be understood that other methods of joining the layers of the assembly 10 may be employed, e.g. using double sided adhesive tape. Stitching may be provided along critical edges or at specific points where additional reinforcement is needed.

The illustrated assembly 10 comprises just a top layer 15, a single absorbent core layer 14 and a moisture barrier layer 12, but it will be understood that any number of additional layers may be provided depending upon requirements. Thus, further core layers may be provided with different sizes and absorbent characteristics. Wicking layers and distribution layers may also be provided. Any layers between the top layer 15 and the barrier layer 12 may be termed intermediate layers.

The top layer 15 comprises a water-permeable material thus allowing the body fluids to migrate to the underlying core layer 14. The top layer 15 may be constructed of any suitable fabric, including naturally derived fibres selected from the group consisting of cotton, wool, silk, cellulose, regenerated cellulose, rayon, viscose, modal, lyocell, Tencel, bamboo, hemp, flax, ramie, coir, or banana. Alternatively, the top layer 15 may be constructed of synthetic fibres selected from the group consisting of polyamide, acrylic, polyester, or elastane, such as a mixture of polyester and elastane. Further, the top layer 15 may be constructed of a blend or a mixture of naturally derived and/or synthetic fibres. The materials used for construction of the top layer 15 should be soft and non-irritating to the skin and be readily penetrated by any body fluids. The top layer 15 is washable and may be of a knitted material. The top layer 15 may have a base weight of 80-200 gsm.

The absorbent core layer 14 is washable and comprises any knitted or woven material capable of absorbing fluid, such as woven or knitted microfibre or polymer, fabric formed from hydrophilic fibres, absorbent fibres. The absorbent core layer 14 may comprise natural or synthetic fibres as described above for the top layer 15 but may alternatively include polyester or polyamide. The core layer 14 may comprise between 20% and 100% natural or naturally derived fibres, such as between 40% and 100% natural or naturally derived fibres and such as between 60% and 100% natural or naturally derived fibres. The basis weight of the core layer 14 may be 180-600 gsm. In the illustrated example, the core layer 14 is a tightly knit interlock knitted fabric having a single-sided terry finish at the wearer facing interior side 8.

The moisture barrier layer 12 is washable and may comprise a film or laminate e.g. of extruded polymeric material or a woven or knitted material of any suitable construction to prevent liquid from migrating from the assembly 10 to the fabric panel 2. The moisture barrier layer 12 in the illustrated variant, is a tightly woven activated carbon cloth that is sufficiently impermeable by nature. One suitable material is Flexzorb ® from Chemviron SA. Laminates with polymer films may also be used. The activated carbon material may be highly desirable in reducing odours. The moisture barrier layer 12 may also comprise a coating of a moisture-impermeable material. The coating may be a polymer such as urethane wax or polyurethane provided for example on the surface facing away from the wearer of the moisture barrier layer 12.

The moisture barrier layer 12 may be breathable, to allow vapour to escape from the absorbent undergarment 1 while preventing liquid from passing through the fabric layer. Air and vapour permeability may be achieved by the woven or knitted material or by an additional rate controlling layer attached thereto.

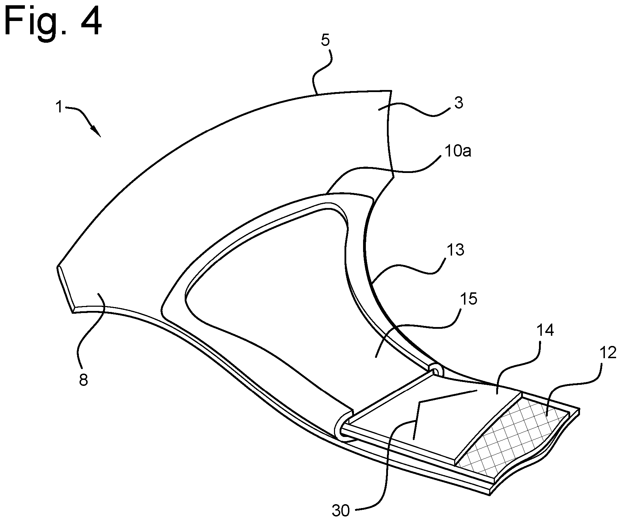

Fig. 4 shows part of the garment 1 in cutaway perspective view, viewed towards an inside surface 8 of the front side 3. The top layer 15 is partially removed to reveal the absorbent core layer 14 and the moisture barrier layer 12 beneath. As can be seen in this view, the core layer 14 is provided with a chevron-shaped incision 30, which serves as a stress-release zone as will be further described below.

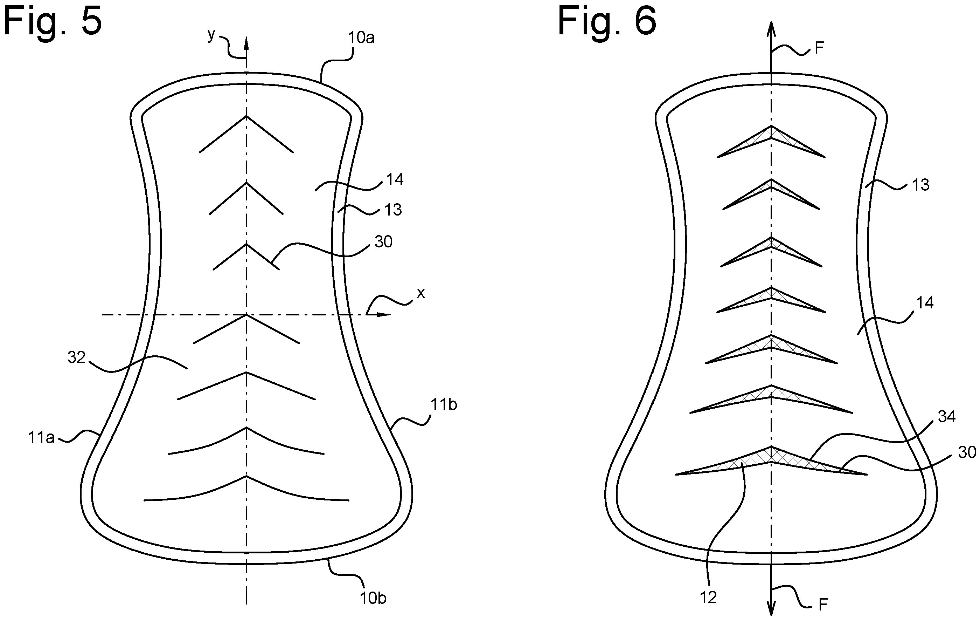

Fig. 5 shows the assembly 10 of Fig. 4 in plan view, viewed towards its inside surface with the top layer 15 removed. The core layer 14 is visible, as is the single sided adhesive tape 13 extending around the side edges 11a, 11b and the front and rear edges 10a, 10b. In this view it can be seen that the core layer 14 is provided with a plurality of chevron-shaped incisions 30 extending generally in the transverse direction x and aligned along the central longitudinal axis y. The incisions 30 partially separate the core 14 into interconnected regions 32 that are joined to each other adjacent the side edges 11a, 11b.

Figure 6 shows the assembly 10 of Fig. 5 under application of a force F along the central longitudinal axis y. As can be seen, the incisions 30 allow the core layer 14 to stretch by forming openings 34, through which the moisture barrier layer 12 is visible beneath. In this manner, stress induced in the core layer 14 by the force F can be relieved.

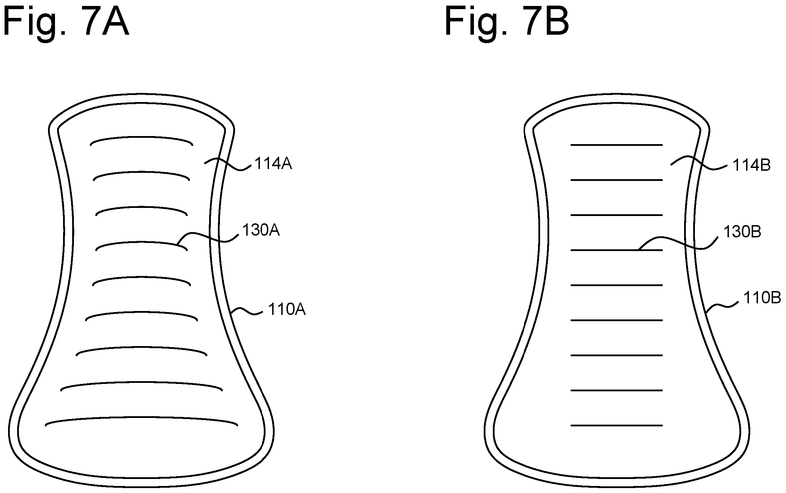

Figures 7 A-D show alternative assemblies 110 A-D in a view corresponding to that of Fig. 5, with different distributions of incisions 130 A-D through the core layer 114 A-D. As in Fig. 5 a top layer has been removed to visualise the core layer.

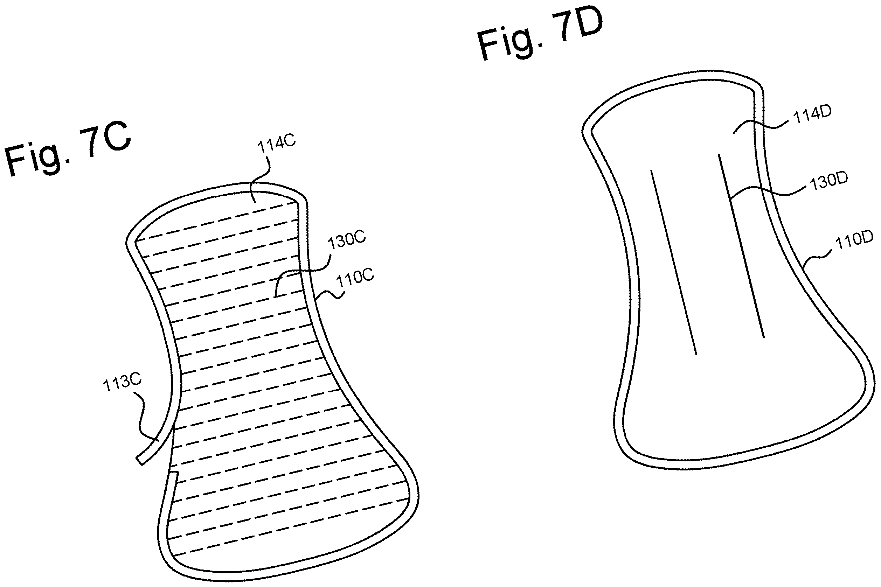

In the case of Fig. 7A, the incisions 130A are generally curved in shape and extend across around 70% of the width of the core layer 114A. Since the assembly 110A is narrower at the crotch than at the front and rear edges, the incisions 130A also vary in length. In the case of Fig. 7B, the incisions 130B are straight and parallel with each other and aligned with the transverse direction X. In this case, the incisions 130B are all the same length. In Fig. 7C, the core layer 114C is provided with a much larger number of incisions 130C than in previous examples. Each incision 130C, extends only across about 10% of the width of the core layer 114C. Nevertheless, multiple incisions 130C are aligned with each other in the transverse direction x. Part of the tape 113C has been removed to show that in this case, the incisions extend to and intercept the side edge of the core layer 114C. In Fig. 7C, the core layer 114C will distort under an applied longitudinal force to form multiple small openings. In Fig. 7D two incisions 130D extend in the longitudinal direction y through the core layer 114D.

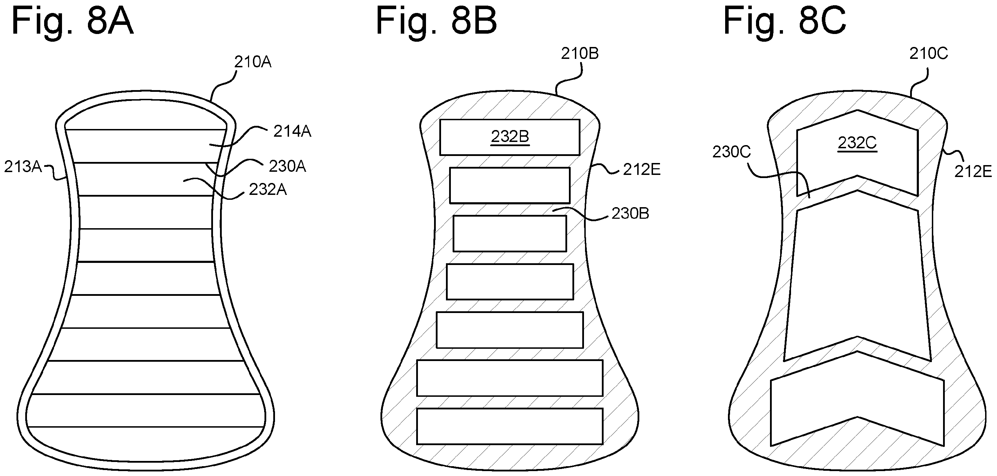

Figures 8A-C show alternative examples of an assembly 210 A-C in a view corresponding to that of Fig. 5, with the core layer 214 A-C divided into segments. As in Fig. 5 a top layer has been removed to visualise the core layer.

In the case of Fig. 8A, the core layer 214A is provided with separations 230A that divide the core layer 214A into segments 232A. The segments 232A extend the full width of the assembly 210A and are retained and connected to a further layer (not shown) by the tape 213A. Adjacent segments 232A are in direct contact with each other although it will be understood that they may separate once a longitudinal force is applied to the assembly 210A.

An alternative arrangement is depicted in Fig. 8B, in which the core layer 214B is divided into a plurality of segments 232B that are separated from each other by gaps 230B. In this case, the individual segments 232B do not extend to the side edges of the assembly and are not held in position by a tape as was the case for previous examples. In Fig. 8B, the segments 232B of the core layer 214B are directly connected to a further layer 214E. The gaps 230B between the segments 232B serve as stress-release zones, which can increase in size as a force is applied to the assembly 210B causing the further core layer 214E, to stretch.

Fig. 8C is a similar arrangement to that of Fig. 8B but the assembly 210C has core layer segments 232C each having a chevron shape, with chevron shaped gaps 230C between them.

**Test Method for Cloth Stretchability**

The following procedure may be used for determining cloth stretchability:

For a representative article, the cloth region under investigation is arranged perfectly flat (but not stretched). Samples are then cut from the directions of interest, preferably using a sharp punching tool. The samples should be representative for the studied cloth region and obvious irregularities (such as seams or fastening elements) should be avoided. A standard sample should be 40 mm wide, and more than 40 mm long (some extra material is required in the length direction for attachment into clamps, as explained further below).

Before testing, the samples are conditioned for at least 24 hours in an environment set to 23°C and 50% relative humidity, and subsequent testing takes place in this same environment.

A tensile tester is utilized for the measurement. Suitable machines are available e.g. from the Instron, Lloyd or Zwick companies. The tester is provided with a lower stationary clamp, vertically aligned with an upper clamp that can be raised at a constant rate (in this instance 300 mm/min). The clamps must be as wide or wider than the tested sample.

The clamps are set 40 mm apart (the sample gauge length). Slipping in the clamps must be prevented, for example by attaching tape with a high friction backing immediately adjacent to the tested 40 mm section (onto the whole sample area that is attached into the clamps). Tape can also be used to extend the sample length to facilitate insertion into the clamps.

The tensile tester is zeroed. The sample is then arranged into the clamps so that it rests vertically and straight (but not stretched).

The test is initiated, and the sample elongation is read when a force of 4 Newton has been reached for the standard sample. Stretchability is then calculated as:

$$(\text{Sample length at 4 N} - \text{Initial sample length}) / \text{Initial sample length} \times 100$$

The result is expressed as percentual stretch at a force of 1 N per cm of sample width.

For example, if a force of 4 N is read when a standard test section (40 mm long and 40 mm wide) has been elongated with 20 mm (the clamps are now 60 mm apart), the elongation at a force of 1 N per cm of sample width is determined to 50%.

In cases where the cloth region of interest is small and a sample of standard dimensions cannot be cut, samples as large as possible should be obtained. In this case, the sample length to width ratio must be maintained, and the reading force adjusted accordingly (e.g. a 30 mm long test section should have a width of 30 mm, and percentual stretch should be read at 3 N).

The disclosure may be varied within the scope of the appended claims. For example, the materials and dimensions used for the different layers forming the absorbent insert may be varied, as indicated above.

**Claims**

1. A washable integrated absorbent assembly (10) for a washable absorbent undergarment (1), the assembly extending in a longitudinal direction and having a transverse direction, the assembly comprising an absorbent core layer of knitted or woven material connected to one or more further layers, wherein the core layer is provided with one or more stress-release zones to facilitate stretching of the core layer.

2. The assembly of claim 1, wherein the core layer comprises a plurality of separate segments and the stress-release zones extend between adjacent segments.

3. The assembly of claim 1 or claim 2, wherein the stress-release zones comprise incisions extending partly across the core layer to divide the core layer into interconnected regions.

4. The assembly of claim 3, wherein each incision extends between 10 % and 90 % across the width of the core layer in the transverse direction.

5. The assembly of claim 3 or claim 4, comprising 1 to 100 incisions.

6. The assembly of any one of the preceding claims, wherein the core layer comprises from 2 to 30 separate segments or partly interconnected regions.

7. The assembly of any one of the preceding claims, wherein the stress-release zones are non-linear.

8. The assembly of any one of the preceding claims, wherein the stress-release zones extend at least partially in the transverse direction to facilitate stretching of the core layer in the longitudinal direction.

9. The assembly of any one of the preceding claims, wherein the further layers comprise a wearer facing top layer and/or a moisture distributing layer and/or further absorbent core layers.

10. The assembly of any one of the preceding claims, wherein the further layers comprise a garment facing moisture barrier layer.

11. The assembly of any one of the preceding claims, wherein the knitted or woven material of the core layer exhibits lower stretchability than any of the one or more further layers.

12. The assembly of any one of the preceding claims, wherein the core layer is connected to the one or more further layers at least partially at its side edges.

13. A washable absorbent undergarment (1), comprising one or more woven or knitted fabric panels (2) and an assembly (10) according to any one of the preceding claims located in a crotch region of the undergarment.

14. The garment according to claim 13, wherein the assembly is permanently attached to at least one of the one or more fabric panels.

15. The garment according to any one of claims 13 or 14, wherein the fabric panels form a front region (3), a back region (4) and a crotch region (7) extending between the front and back regions (3, 4), the front region (3) and the back region (4) being joined such that the undergarment (1) forms a waist opening (5) and a pair of leg openings (6a, 6b), wherein a central longitudinal axis (y) of the undergarment (1) is defined along the one or more fabric panels (2) of the undergarment (1) from the back region (4) and towards the front region (3), and a transversal crotch axis (x) of the undergarment (1) is defined in a direction extending between the leg openings (6a, 6b) and so as to divide the crotch region (7) into a front crotch region (7a) extending longitudinally between the transversal crotch axis (x) and a front end (61a, 61b) of each leg opening (6a, 6b), and a rear crotch region (7b) extending longitudinally between the transversal crotch axis (x) and a rear end (62a, 62b) of each leg opening (6a, 6b), the transversal crotch axis (x) being perpendicular to the central longitudinal axis (y).

## Fig. 1

## Fig. 1A

# Fig. 2

# Fig. 3

# Fig. 4

Fig. 5

Fig. 6

# Fig. 7A

114A

130A

110A

# Fig. 7B

114B

130B

110B

EP 4 736 827 A1

Fig. 7D

114D

130D

110D

Fig. 7C

114C

130C

110C

113C

Fig. 8A

Fig. 8B

Fig. 8C

**EUROPEAN SEARCH REPORT**

Application Number

EP 24 21 0515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 2 622 001 A (WUKA LTD [GB]) 6 March 2024 (2024-03-06) | 1,2,6-15 | INV. A61F13/505 |
| A | * claims 1-3; figures 3,5A,5B * ----- | 3-5 | A61F13/49 A61F13/533 |
| A | AU 2022 445 925 B2 (ESSITY HYGIENE & HEALTH AB [SE]) 3 October 2024 (2024-10-03) * figures 1-3 * ----- | 1-15 | A61F13/534 A61F13/53 |
| A | WO 2024/165154 A1 (ESSITY HYGIENE & HEALTH AB [SE]) 15 August 2024 (2024-08-15) * paragraph [0050]; claim 1 * ----- | 1-15 | |
| A | US 6 969 378 B1 (VUKOS JOHN PHILIP [US] ET AL) 29 November 2005 (2005-11-29) * figure 4 * ----- | 1-15 | |

**TECHNICAL FIELDS SEARCHED (IPC)**

A61F

The present search report has been drawn up for all claims

| Place of search | Date of completion of the search | Examiner |
|---|---|---|
| Munich | 26 March 2025 | Beins, Ulrika |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 21 0515

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2025

| Patent document cited in search report | | Publication date | Patent family member(s) | | Publication date |
|---|---|---|---|---|---|
| GB 2622001 | A | 06-03-2024 | GB | 2622001 A | 06-03-2024 |
| | | | WO | 2024047053 A1 | 07-03-2024 |
| AU 2022445925 | B2 | 03-10-2024 | AU | 2022445925 A1 | 08-08-2024 |
| | | | CN | 118785876 A | 15-10-2024 |
| | | | CO | 2024012636 A2 | 30-09-2024 |
| | | | EP | 4489709 A1 | 15-01-2025 |
| | | | WO | 2023169665 A1 | 14-09-2023 |
| WO 2024165154 | A1 | 15-08-2024 | NONE | | |
| US 6969378 | B1 | 29-11-2005 | AR | 031053 A1 | 03-09-2003 |
| | | | AU | 9658201 A | 06-05-2002 |
| | | | EP | 1330222 A1 | 30-07-2003 |
| | | | JP | 4564712 B2 | 20-10-2010 |
| | | | JP | 2004525661 A | 26-08-2004 |
| | | | KR | 20030044053 A | 02-06-2003 |
| | | | MA | 25845 A1 | 01-07-2003 |
| | | | MX | PA03003520 A | 07-08-2003 |
| | | | PL | 365852 A1 | 10-01-2005 |
| | | | US | 6969378 B1 | 29-11-2005 |
| | | | WO | 0234184 A1 | 02-05-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023169665 A1